# EUROPÄISCHE PATENTSCHRIFT

(11) **EP 0 776 889 B1**
(45) Veröffentlichungstag und Bekanntmachung des Hinweises auf die Patenterteilung: **06.03.2002**
(21) Anmeldenummer: 96118553.5
(22) Anmeldetag: 20.11.1996
(51) Int. Cl.: C07D 231/14

(54) **Verfahren zur Herstellung von 1,3-Dimethyl-5-fluor-pyrazol-4-carboxaniliden**
Process for the production of 1,3-dimethyl-5-fluoro-pyrazol-4-carboxanilides
Procédé pour la préparation de 1,3-diméthyl-5-fluoro-pyrazol-4-carboxanilides

(30) Priorität: 01.12.1995 DE 19544800
(43) Veröffentlichungstag der Anmeldung: 04.06.1997
(73) Patentinhaber: BAYER AG, 51368 Leverkusen (DE)
(72) Erfinder: Gallenkamp, Bernd, Dr., 42113 Wuppertal (DE); Rohe, Lothar, Dr., 42113 Wuppertal (DE); Marhold, Albrecht, Dr., 51375 Leverkusen (DE)

(56) Entgegenhaltungen:
- DE-C- 665 598
- SYNTHETIC COMMUNICATIONS, Bd. 12, Nr. 7, 1982, Seiten 513-520, XP000651185 M. TORDEUX, C. WAKSELMAN: "Reaction of potassium fluoride in the presence of tetraalkylammonium halides. Preparation of acylfluorides and fluoroformates"
- JOURNAL OF HETEROCYCLIC CHEMISTRY, Bd. 22, 1985, Seiten 1621-1630, XP000650285 L.F. LEE ET AL.: "Syntheses and Reactions of 2-Halo-5-thiazolecarboxylates"
- JOURNAL OF FLUORINE CHEMISTRY, Bd. 59, Nr. 1, 1992, Seiten 15-31, XP000651189 H.C. FIELDING, I.M. SHIRLEY: "Synthesis and reactions of 4-sulpho-2,3,5,6-tetrafluorobenzoic acid"

## Beschreibung

Die vorliegende Erfindung betrifft ein neues Verfahren zur Herstellung von 1,3-Dimethyl-5-fluor-pyrazol-4-carboxaniliden, die als Wirkstoffe mit fungiziden Eigenschaften bekannt sind. Außerdem betrifft die Erfindung auch das neue 1,3-Dimethyl-5-fluor-pyrazol-4-carbonsäure-fluorid und dessen Verwendung als Zwischenprodukt.

Es ist bereits bekannt geworden, daß bestimmte 1,3-Dimethyl-5-fluor-pyrazol-4-carboxamide zugänglich sind, indem man die entsprechenden 5-Chlor-pyrazol-Derivate fluoriert (vgl. EP-A 0 199 822). So erhält man durch Behandlung von 1,3-Dimethyl-N-(1,1-dimethyl-indan-4-yl)-5-chlor-pyrazol-4-carbonsäureamid mit Kaliumfluorid das 1,3-Dimethyl-N-(1,1-dimethyl-indan-4-yl)-5-fluor-pyrazol-4-carbonsäureamid. Nachteilig an diesem Verfahren ist, daß ein durch aufwendige Synthese hergestelltes Produkt als Ausgangsstoff eingesetzt wird. Ungünstig ist auch, daß die Ausbeuten an den gewünschten Verbindungen relativ niedrig liegen. Da außerdem hohe Reaktionstemperaturen erforderlich sind, lassen sich auf diese Weise nur Substanzen mit hitzebeständigen Substituenten herstellen.

Weiterhin ist schon bekannt geworden, daß sich zahlreiche 1-Methyl-5-fluorpyrazol-4-carboxanilide ausgehend von den entsprechenden 1-Methyl-5-chlorpyrazol-4-carbaldehyden synthetisieren lassen (vgl. WO 93-11 117). So erhält man z.B. das 1,3-Dimethyl-5-fluor-pyrazol-4-carbonsäure-(2-cycloheptyl)-anilid, indem man 1,3-Dimethyl-5-chlor-pyrazol-4-carbaldehyd mit Kaliumfluorid umsetzt, den dabei entstehenden 1,3-Dimethyl-5-fluor-pyrazol-4-carbaldehyd mit Kaliumdichromat in die 1,3-Dimethyl-5-fluor-pyrazol-4-carbonsäure überführt, aus dieser Verbindung durch Behandlung mit Thionylchlorid das 1,3-Dimethyl-5-fluorpyrazol-4-carbonsäure-chlorid bereitet und letzteres mit 2-Cycloheptyl-anilin zur Reaktion bringt. Diese Synthese kann durch das folgende Formelschema veranschaulicht werden:

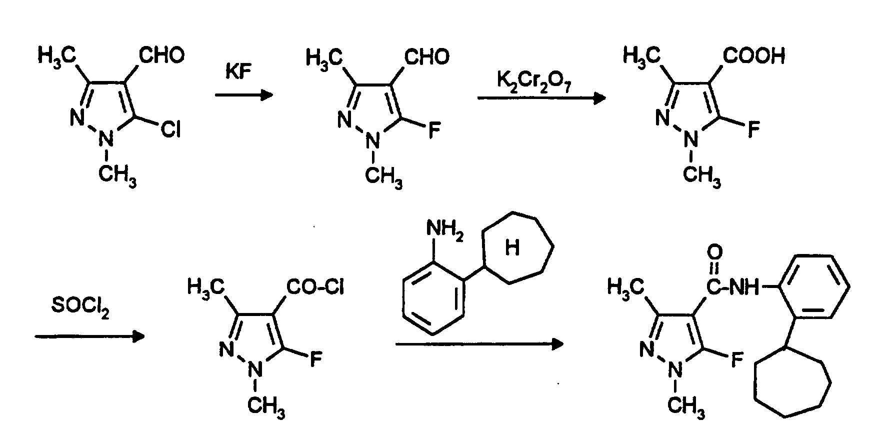

Ein entscheidender Nachteil dieses Verfahrens besteht darin, daß viele Reaktionsschritte erforderlich sind und die Fluorierung in der ersten Stufe in sehr niedrigen Ausbeuten verläuft.

Es wurde nun gefunden, daß man 1,3-Dimethyl-5-fluor-pyrazol-4-carboxanilide der Formel 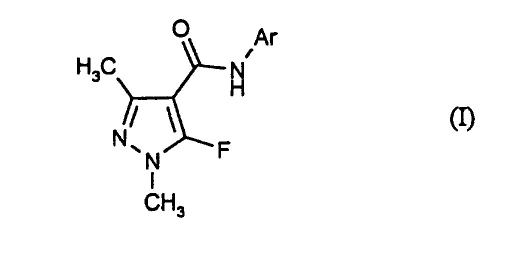 in welcher
- Ar: für gegebenenfalls substituiertes Phenyl steht,
erhält, wenn man
a) in einer ersten Stufe 1,3-Dimethyl-5-chlor-pyrazol-4-carbonsäure-chlorid der Formel 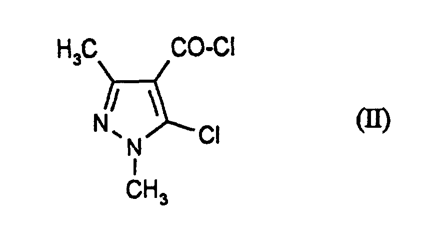 mit Fluoriden gegebenenfalls in Gegenwart eines Verdünnungsmittels umsetzt und
b) in einer zweiten Stufe das dabei entstehende 1,3-Dimethyl-5-fluor-pyrazol-4-carbonsäure-fluorid der Formel 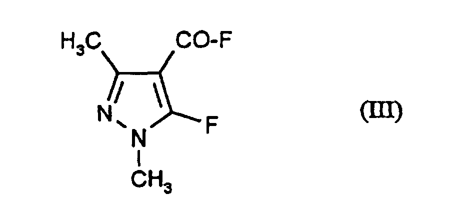 mit Anilinen der Formel

   H₂N-Ar (IV)

   in welcher
   - Ar: die oben angegebene Bedeutung hat,
gegebenenfalls in Gegenwart eines Säureakzeptors und gegebenenfalls in Gegenwart eines Verdünnungsmittels umsetzt.

Es ist als äußerst überraschend zu bezeichnen, daß sich 1,3-Dimethyl-5-fluorpyrazol-4-carboxanilide nach dem erfindungsgemäßen Verfahren in hoher Ausbeute und ausgezeichneter Reinheit herstellen lassen. Aufgrund des bekannten Standes der Technik war nämlich damit zu rechnen, daß die Fluorierung am Pyrazol-Ring und in der Säure-Gruppe nicht gleichermaßen gut gelingen würden. Außerdem war anzunehmen, daß das Säurefluorid bei der Umsetzung mit Anilinen weniger reaktiv ist als das entsprechende Säurechlorid und die Bildung der gewünschten Endprodukte deshalb auf Schwierigkeiten stoßen würde. Im Gegensatz zu den Erwartungen ist das aber nicht der Fall. Das zweistufige Verfahren verläuft vielmehr problemlos und ohne nennenswerte Nebenreaktionen.

Setzt man 1,3-Dimethyl-5-chlor-pyrazol-4-carbonsäure-chlorid in der ersten Stufe mit Kaliumfluorid um und läßt man das dabei entstehende 1,3-Dimethyl-5-fluorpyrazol-4-carbonsäure-fluorid in der zweiten Stufe mit 2-Cycloheptyl-anilin reagieren, so kann der Verlauf des erfindungsgemäßen Verfahrens durch das folgende Formelschema veranschaulicht werden:

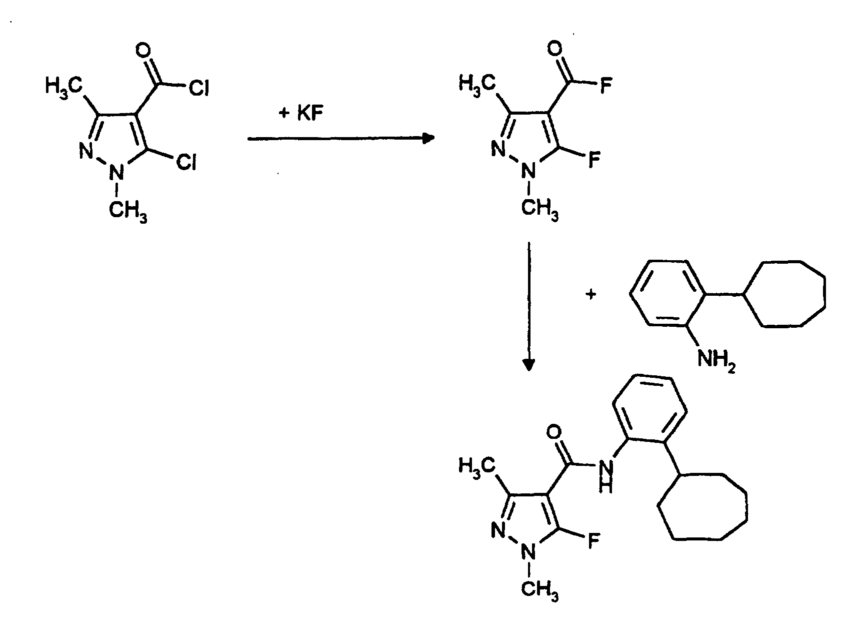

Das bei der Durchführung des erfindungsgemäßen Verfahrens als Ausgangsstoff benötigte 1,3-Dimethyl-5-chlor-pyrazol-4-carbonsäure-chlorid der Formel (II) ist bekannt (vgl. JP-A 1990 - 85 257 und Chem. Abstr. 113, 78 387).

Als Reaktionskomponenten kommen bei der Durchführung der ersten Stufe des erfindungsgemäßen Verfahrens alle üblichen Metallfluoride, Ammoniumfluorid und Phosphoniumfluoride in Frage. Vorzugsweise verwendbar sind Alkalimetallfluoride, wie Kaliumfluorid und Cäsiumfluorid, ferner Ammoniumfluorid oder Triphenylmethylphosphonium-fluorid. Die Fluoride sind bekannt.

Das bei der Durchführung der ersten Stufe des erfindungsgemäßen Verfahrens entstehende 1,3-Dimethyl-5-fluor-pyrazol-4-carbonsäure-fluorid der Formel (III) ist neu. Es dient als Ausgangsstoff für die Durchführung der zweiten Stufe des erfindungsgemäßen Verfahrens.

Die zur Durchführung der zweiten Stufe des erfindungsgemäßen Verfahrens als Reaktionskomponenten benötigten Aniline sind durch die Formel (IV) allgemein definiert. In dieser Formel steht
- Ar: vorzugsweise für eine Gruppierung der Formel 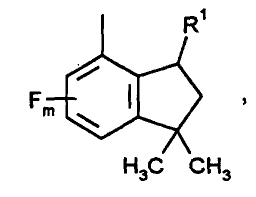 in welcher
m für 0 oder 1 steht und
R¹ für Wasserstoff oder Methyl steht,
oder
- Ar: steht vorzugsweise für eine Gruppierung der Formel 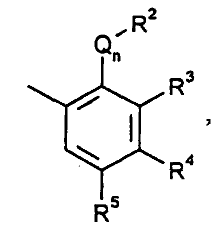 in welcher
n für 0 oder 1 steht,
Q für Alkylen mit 1 bis 3 Kohlenstoffatomen, Alkenylen mit 2 oder 3 Kohlenstoffatomen oder Alkinylen mit 2 oder 3 Kohlenstoffatomen steht oder für eine Gruppierung der Formel -(CH₂)ₜ-CH= steht, in welcher
t für die Zahlen 0, 1, 2 oder 3 steht und die -(CH₂)ₜ-Einheit mit dem Phenylring verbunden ist, oder für eine Gruppierung der Formel -(CH₂)ₚ-Y-(CH₂)ᵣ- steht, in welcher
p und r unabhängig voneinander für die Zahlen 0, 1 oder 2 stehen und
Y für Sauerstoff oder Schwefel steht, wobei jeweils die -(CH₂)ₚ-Einheit mit dem Phenylring verbunden ist,
R² für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Cycloalkyl mit 3 bis 12 Kohlenstoffatomen steht, oder für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Cycloalkenyl mit 3 bis 12 Kohlenstoffatomen steht, oder
für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Bicycloalkyl mit 6 bis 12 Kohlenstoffatomen steht, oder
für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Oxacycloalkyl mit 2 bis 12 Kohlenstoffatomen steht, oder
für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Oxacycloalkenyl mit 4 bis 12 Kohlenstoffatomen steht, oder
für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Thiacycloalkyl mit 3 bis 12 Kohlenstoffatomen steht, oder
für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Thiacycloalkenyl mit 4 bis 12 Kohlenstoffatomen steht, oder
für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Azacycloalkyl mit 2 bis 12 Kohlenstoffatomen steht,
wobei der gesättigte oder ungesättigte Ring jeweils zweifach gebunden ist, wenn -Q-R² für -(CH₂)ₜ-CH= steht, und
R³, R⁴ und R⁵ unabhängig voneinander für Wasserstoff, Halogen, Cyano, Alkyl mit 1 bis 6 Kohlenstoffatomen, Alkenyl mit 2 bis 6 Kohlenstoffatomen, Alkinyl mit 2 bis 6 Kohlenstoffatomen, Alkoxy mit 1 bis 4 Kohlenstoffatomen, Alkylthio mit 1 bis 4 Kohlenstoffatomen, Cycloalkyl mit 3 bis 6 Kohlenstoffatomen oder Halogenmethoxy stehen.
- Ar: steht auch besonders bevorzugt für eine Gruppierung der Formel 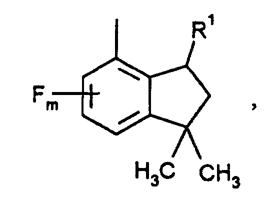 in welcher
m für 0 oder 1 steht und
R¹ für Wasserstoff oder Methyl steht.
Außerdem steht
- Ar: auch besonders bevorzugt für eine Gruppierung der Formel 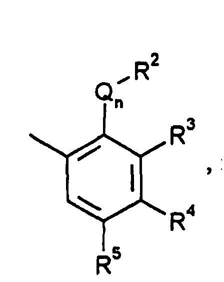 in welcher
- Q: vorzugsweise für geradkettiges oder verzweigtes Alkylen mit 1 bis 3 Kohlenstoffatomen, Alkenylen mit 2 oder 3 Kohlenstoffatomen, Alkinylen mit 2 oder 3 Kohlenstoffatomen oder für eine Gruppierung der Formel -(CH₂)ₜ-CH= steht, in welcher
t vorzugsweise für die Zahlen 0, 1 oder 2 steht, wobei jeweils die -(CH₂)ₜ-Einheit mit dem Phenylring verbunden ist, oder für eine Gruppierung der Formel -(CH₂)ₚ-Y-(CH₂)ᵣ- steht, in welcher
p und r unabhängig voneinander für die Zahlen 0, 1 oder 2 stehen und
Y für Sauerstoff oder Schwefel steht,
wobei jeweils die -(CH₂)ₚ-Einheit mit dem Phenylring verbunden ist,
n vorzugsweise für die Zahlen 0 oder 1 steht,
R² vorzugsweise für gegebenenfalls einfach bis vierfach, gleichartig oder verschieden durch Alkyl mit 1 bis 4 Kohlenstoffatomen, Alkoxy mit 1 bis 4 Kohlenstoffatomen, Fluor, Chlor, Brom und/oder Cyano substituiertes Cycloalkyl mit 3 bis 8 Kohlenstoffatomen steht, oder
für gegebenenfalls einfach bis vierfach, gleichartig oder verschieden durch Alkyl mit 1 bis 4 Kohlenstoffatomen, Alkoxy mit 1 bis 4 Kohlenstoffatomen, Fluor, Chlor, Brom und/oder Cyano substituiertes Cycloalkenyl mit 3 bis 8 Kohlenstoffatomen steht, oder
für gegebenenfalls einfach bis dreifach, gleichartig oder verschieden durch Alkyl mit 1 bis 4 Kohlenstoffatomen, Alkoxy mit 1 bis 4 Kohlenstoffatomen, Fluor, Chlor, Brom und/oder Cyano substituiertes Bicycloalkyl mit 6 bis 10 Kohlenstoffatomen steht, oder
für gegebenenfalls einfach bis dreifach, gleichartig oder verschieden durch Alkyl mit 1 bis 4 Kohlenstoffatomen, Alkoxy mit 1 bis 4 Kohlenstoffatomen, Fluor, Chlor, Brom und/oder Cyano substituiertes Oxacycloalkyl mit 2 bis 7 Kohlenstoffatomen steht, oder
für gegebenenfalls einfach bis dreifach, gleichartig oder verschieden durch Alkyl mit 1 bis 4 Kohlenstoffatomen, Alkoxy mit 1 bis 4 Kohlenstoffatomen, Fluor, Chlor, Brom und/oder Cyano substituiertes Oxacycloalkenyl mit 4 bis 7 Kohlenstoffatomen steht, oder
für gegebenenfalls einfach bis dreifach, gleichartig oder verschieden durch Alkyl mit 1 bis 4 Kohlenstoffatomen, Alkoxy mit 1 bis 4 Kohlenstoffatomen, Fluor, Chlor, Brom und/oder Cyano substituiertes Thiacycloalkyl mit 3 bis 7 Kohlenstoffatomen steht, oder
für gegebenenfalls einfach bis dreifach, gleichartig oder verschieden durch Alkyl mit 1 bis 4 Kohlenstoffatomen, Alkoxy mit 1 bis 4 Kohlenstoffatomen, Fluor, Chlor, Brom und/oder Cyano substituiertes Thiacycloalkenyl mit 4 bis 7 Kohlenstoffatomen steht, oder
für gegebenenfalls einfach bis dreifach, gleichartig oder verschieden durch Alkyl mit 1 bis 4 Kohlenstoffatomen, Alkoxy mit 1 bis 4 Kohlenstoffatomen, Fluor, Chlor, Brom und/oder Cyano substituiertes Azacycloalkyl mit 2 bis 7 Kohlenstoffatomen steht,
wobei der Ring in den zuvor genannten Resten jeweils zweifach gebunden ist, wenn die Gruppierung -Q-R² für -(CH₂)ₜ-CH= steht,
R³, R⁴ und R⁵ unabhängig voneinander für Wasserstoff, Fluor, Chlor, Brom, Cyano, Alkyl mit 1 bis 4 Kohlenstoffatomen, Alkenyl mit 2 bis 4 Kohlenstoffatomen, Alkinyl mit 2 bis 4 Kohlenstoffatomen, Methoxy, Ethoxy, Methylthio, Ethylthio, Cyclopropyl, Cyclopentyl, Cyclohexyl, Fluormethoxy, Difluormethoxy, Trifluormethoxy, Chlormethoxy, Dichlormethoxy oder Trichlormethoxy stehen.
- Ar: steht ganz besonders bevorzugt für eine Gruppierung der Formel 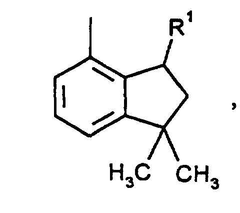 in welcher
R¹ für Wasserstoff oder Methyl steht.
- Ar: steht außerdem ganz besonders bevorzugt für eine Gruppierung der Formel 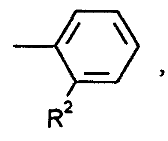 in welcher
R² für Cyclopentyl, Cyclohexyl, Cycloheptyl oder Bicyclo[2,2,1]-heptyl steht.

Die Aniline der Formel (IV) sind bekannt oder lassen sich nach bekannten Methoden herstellen (vergleiche WO 93-11 117).

Als Verdünnungsmittel zur Durchführung der ersten Stufe des erfindungsgemäßen Verfahrens kommen alle polaren, aprotischen organischen Lösungsmittel in Betracht. Vorzugsweise verwendbar sind Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol, ferner Ketone, wie Aceton, Butanon, Methyl-isobutylketon oder Cyclohexanon, außerdem Nitrile, wie Acetonitril, Propionitril, n- oder i-Butyronitril oder Benzonitril; weiterhin Amide, wie N,N-Dimethylformamid, N,N-Dimethylacetamid, N-Methylformanilid, N-Methylpyrrolidon oder Hexamethylphosphorsäuretriamid; ferner Ester, wie Essigsäuremethylester oder Essigsäureethylester und auch Sulfoxide, wie Dimethylsulfoxid, oder Sulfone, wie Sulfolan.

Als besonders bevorzugte Verdünnungsmittel kommen Sulfone, wie Sulfolan, in Betracht.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen 100°C und 250°C, vorzugsweise zwischen 150°C und 200°C.

Sowohl bei der Durchführung der ersten als auch der zweiten Stufe des erfindungsgemäßen Verfahrens arbeitet man im allgemeinen unter Normaldruck. Es ist jedoch auch möglich, unter erhöhtem oder vermindertem Druck zu arbeiten. Dabei arbeitet man im allgemeinen unter Drucken zwischen 0,1 bar und 10 bar.

Als Säureakzeptoren kommen bei der Durchführung der zweiten Stufe des erfindungsgemäßen Verfahrens alle üblichen anorganischen und organischen Säurebindemittel in Frage. Vorzugsweise verwendbar sind Erdalkalimetall- oder Alkalimetallhydroxide, -carbonate oder -hydrogencarbonate, wie beispielsweise Natriumhydroxid, Kaliumhydroxid, Natriumcarbonat, Kaliumcarbonat, Kaliumhydrogencarbonat oder Natriumhydrogencarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Besonders bevorzugt verwendbar sind bicyclische tertiäre Amine, wie Diazabicyclooctan (DABCO), Diazabicyclononen (DBN) oder Diazabicycloundecen (DBU).

Als Verdünnungsmittel kommen bei der Durchführung der zweiten Stufe des erfindungsgemäßen Verfahrens alle inerten organischen Lösungsmittel in Betracht. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin, ferner halogenierte Kohlenwasserstoffe, wie Chlorbenzol, Dichlorbenzol, Dichlormethan, Chloroform, Tetrachlormethan, Dichlorethan oder Trichlorethan, außerdem Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; weiterhin Ketone, wie Aceton, Butanon, Methyl-isobutylketon oder Cyclohexanon, darüber hinaus Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid, ferner Ester wie Essigsäuremethylester oder Essigsäureethylester; Sulfoxide, wie Dimethylsulfoxid oder Sulfone, wie Sulfolan.

Als besonders bevorzugte Verdünnungsmittel kommen Nitrile, wie Acetonitril, Propionitril, n- oder i-Butyronitril in Betracht.

Die Reaktionstemperaturen können auch bei der Durchführung der zweiten Stufe des erfindungsgemäßen Verfahrens in einem größeren Bereich variiert werden. Im allgemeinen arbeitet man bei Temperaturen zwischen 0°C und 120°C, vorzugsweise zwischen 50°C und 110°C.

Bei der Durchführung der ersten Stufe des erfindungsgemäßen Verfahrens setzt man auf 1 mol an 1,3-Dimethyl-5-chlor-pyrazol-4-carbonsäure-chlorid der Formel (II) im allgemeinen 2 bis 15 mol, vorzugsweise 2 bis 4 mol an Fluorid ein. Im einzelnen geht man im allgemeinen so vor, daß man eine Lösung von Fluorid in einem Solvens mit 1,3-Dimethyl-5-chlor-pyrazol-4-carbonsäure-chlorid versetzt und das entstehende Gemisch bis zur Beendigung der Umsetzung auf die gewünschte Temperatur erhitzt. Die anschließende Aufarbeitung erfolgt nach üblichen Methoden. Im allgemeinen verfährt man in der Weise, daß man das Reaktionsgemisch einer fraktionierten Destillation unter vermindertem Druck unterwirft.

Bei der Durchführung der zweiten Stufe des erfindungsgemäßen Verfahrens setzt man auf 1 mol an 1,3-Dimethyl-5-fluorpyrazol-4-carbonsäure-fluorid der Formel (III) im allgemeinen 1 bis 15 mol, vorzugsweise 1 bis 2 mol an Anilin der Formel (IV) ein. Im einzelnen geht man im allgemeinen so vor, daß man die Komponenten in beliebiger Reihenfolge vermischt und das entstehende Gemisch bis zur Beendigung der Umsetzung bei der gewünschten Temperatur rührt. Die Aufarbeitung erfolgt nach üblichen Methoden. Im allgemeinen verfährt man in der Weise, daß man das Reaktionsgemisch einengt, den verbleibenden Rückstand mit Wasser verrührt, das ausfallende Festprodukt absaugt und gegebenenfalls nach vorherigem Waschen trocknet.

Die erfindungsgemäß herstellbaren 1,3-Dimethyl-5-fluor-pyrazol-4-carboxanilide sind als Wirkstoffe mit fungiziden Eigenschaften bekannt (vgl. WO 93-11 117).

Die Durchführung des erfindungsgemäßen Verfahrens wird durch die folgenden Beispiele veranschaulicht.

### Herstellungsbeispiele:

### Beispiel 1:

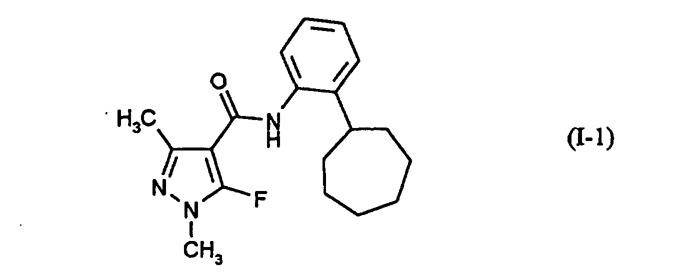

### 1 Stufe

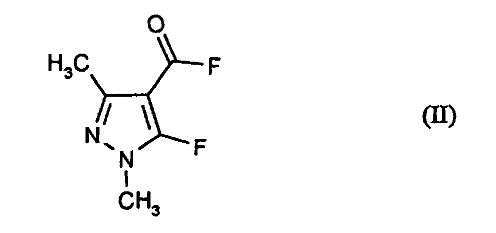

Aus einer Lösung von 437,5 g (7,5 mol) Kaliumfluorid in 3750 ml Sulfolan werden bei einer Temperatur von 190°C unter vermindertem Druck 250 ml Flüssigkeit abdestilliert. Nach dem Abkühlen der Lösung auf Raumtemperatur gibt man 482,6 g (2,5 mol) 1,3-Dimethyl-5-chlor-pyrazol-4-carbonsäure-chlorid hinzu und erhitzt das Gemisch unter Rühren 11 Stunden auf 190°C. Zur anschließenden Aufarbeitung wird das Reaktionsgemisch bei einer Badtemperatur-von 120°C unter einem Druck von 1 mbar über eine 30 cm lange Vigreux-Kolonne fraktioniert destilliert. Als erste Fraktion erhält man dabei 338,7 g (82,1 % der Theorie) an 1,3-Dimethyl-5-fluor-pyrazol-4-carbonsäure-fluorid in Form einer Flüssigkeit, die bei einem Druck von 1 mbar einen Siedepunkt von 68°C aufweist.

### 2 Stufe

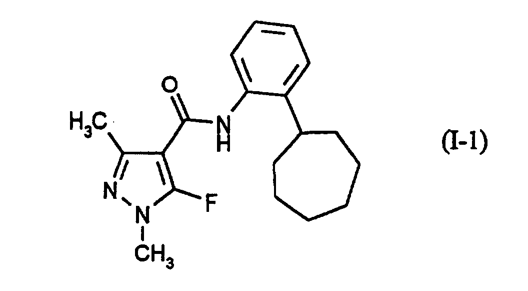

Zu einer Lösung von 264 g (1,26 mol) 1,3-Dimethyl-5-fluorpyrazol-4-carbonsäurefluorid und 245,6 g (1,26 mol) 2-Cycloheptyl-anilin in 2500 ml Acetonitril gibt man unter Rühren 211,7 g Diazabicyclooctan und rührt weitere 17 Stunden bei 70°C. Anschließend wird das Lösungsmittel unter vermindertem Druck abdestilliert, und der verbleibende Rückstand wird mit 1500 ml Wasser verrührt. Der dabei ausfallende Feststoff wird abgesaugt, mit 2000 ml Wasser nachgewaschen und durch weiteres Absaugen getrocknet. Das Produkt wird noch einmal mit 1000 ml Cyclohexan gewaschen und dann bei 50°C unter vermindertem Druck getrocknet. Man erhält auf diese Weise 339,1 g (80,9 % der Theorie) an 1,3-Dimethyl-5-fluor-pyrazol-4-carbonsäure-(2-cycloheptyl)-anilid in Form einer Festsubstanz vom Schmelzpunkt 146°C.

### Vergleichsbeispiel

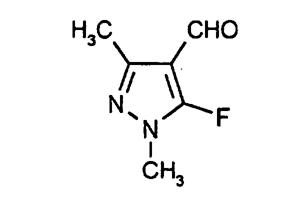

Aus einer Lösung von 6,6 g (0,114 Mol) Kaliumfluorid in 100 ml Sulfolan wird bei einer Temperatur von 150°C unter einem Druck von 20 mbar 30 Minuten lang Flüssigkeit abdestilliert. Nach dem Abkühlen der Lösung auf Raumtemperatur gibt man 12,0 g (0,076 Mol) 1,3-Dimethyl-5-chlor-pyrazol-4-carbaldehyd hinzu und erhitzt das Gemisch unter Rühren 5 Stunden auf 190°C. Zur anschließenden Aufarbeitung wird das Reaktionsgemisch bei einer Badtemperatur von 120°C unter einem Druck von 0,4 mbar über eine 30 cm lange Vigreux-Kolonne fraktioniert destilliert. Als erste Fraktion erhält man dabei 25,6 g einer Flüssigkeit, die bei einem Druck von 0,4 mbar einen Siedebereich von 78 bis 90°C aufweist. Gemäß gaschromatographischer Analyse besteht das Destillat zu 8,2 % aus 1,3-Dimethyl-5-chlor-pyrazol-4-carbaldehyd, zu 80 % aus Sulfolan und zu 10,2 % aus 1,3-Dimethyl-5-fluor-pyrazol-4-carbaldehyd. Danach errechnet sich die Ausbeute an 1,3-Dimethyl-5-fluor-pyrazol-4-carbaldehyd zu 24,2 % der Theorie.

## Patentansprüche

1. Verfahren zur Herstellung von 1,3-Dimethyl-5-fluor-pyrazol-4-carboxaniliden der Formel 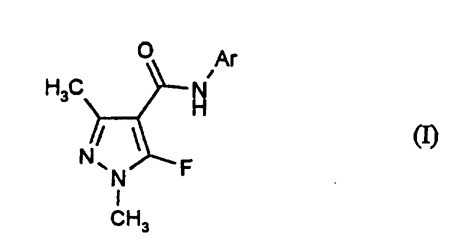 in welcher
Ar für gegebenenfalls substituiertes Phenyl steht,
**dadurch gekennzeichnet, daß** man
a) in einer ersten Stufe 1,3-Dimethyl-5-chlor-pyrazol-4-carbonsäurechlorid der Formel 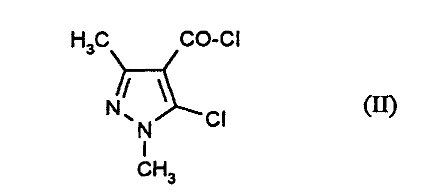 mit Fluoriden gegebenenfalls in Gegenwart eines Verdünnungsmittels umsetzt und
b) in einer zweiten Stufe das dabei entstehende 1,3-Dimethyl-5-fluorpyrazol-4-carbonsäure-fluorid der Formel 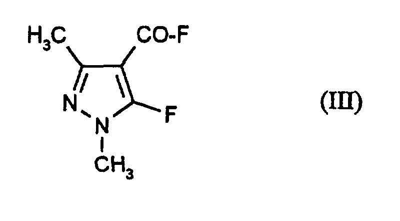 mit Anilinen der Formel
H₂N-Ar (IV)
in welcher
Ar die oben angegebene Bedeutung hat,
gegebenenfalls in Gegenwart eines Säureakzeptors und gegebenenfalls in Gegenwart eines Verdünnungsmittels umsetzt.

2. Verfahren gemäß Anspruch 1, **dadurch gekennzeichnet, daß** man bei der Durchführung der ersten Stufe Alkalimetallfluoride, Ammoniumfluorid oder Phosphoniumfluoride einsetzt.

3. Verfahren gemäß Anspruch 1, **dadurch gekennzeichnet, daß** man bei der Durchführung der zweiten Stufe Aniline der Formel (IV) einsetzt, in denen
Ar für eine Gruppierung der Formel 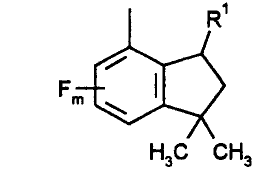 steht, in welcher
m für 0 oder 1 steht und
R¹ für Wasserstoff oder Methyl steht,
oder
Ar für eine Gruppierung der Formel 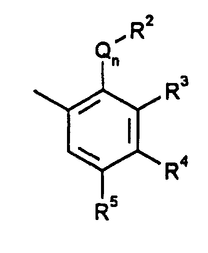 steht, in welcher
n für 0 oder 1 steht,
Q für Alkylen mit 1 bis 3 Kohlenstoffatomen, Alkenylen mit 2 oder 3 Kohlenstoffatomen oder Alkinylen mit 2 oder 3 Kohlenstoffatomen steht oder für eine Gruppierung der Formel -(CH₂)ₜ-CH= steht, in welcher
t für die Zahlen 0, 1, 2 oder 3 steht und die -(CH₂)ₜ-Einheit mit dem Phenylring verbunden ist,
oder für eine Gruppierung der Formel -(CH₂)ₚ-Y-(CH₂)ᵣ- steht, in welcher
p und r unabhängig voneinander für die Zahlen 0, 1 oder 2 stehen und
Y für Sauerstoff oder Schwefel steht, wobei jeweils die -(CH₂)ₚ-Einheit mit dem Phenylring verbunden ist,
R² für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Cycloalkyl mit 3 bis 12 Kohlenstoffatomen steht, oder für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Cycloalkenyl mit 3 bis 12 Kohlenstoffatomen steht,
oder für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Bicycloalkyl mit 6 bis 12 Kohlenstoffatomen steht, oder
für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Oxacycloalkyl mit 2 bis 12 Kohlenstoffatomen steht, oder
für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Oxacycloalkenyl mit 4 bis 12 Kohlenstoffatomen steht, oder
für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Thiacycloalkyl mit 3 bis 12 Kohlenstoffatomen steht, oder
für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Thiacycloalkenyl mit 4 bis 12 Kohlenstoffatomen steht, oder
für gegebenenfalls einfach oder mehrfach, gleichartig oder verschieden durch Alkyl mit 1 bis 8 Kohlenstoffatomen, Alkoxy mit 1 bis 8 Kohlenstoffatomen, Halogen und/oder Cyano substituiertes Azacycloalkyl mit 2 bis 12 Kohlenstoffatomen steht,
wobei der gesättigte oder ungesättigte Ring jeweils zweifach gebunden ist, wenn -Q-R² für -(CH₂)ₜ-CH= steht,
und
R³, R⁴ und R⁵ unabhängig voneinander für Wasserstoff, Halogen, Cyano, Alkyl mit 1 bis 6 Kohlenstoffatomen, Alkenyl mit 2 bis 6 Kohlenstoffatomen, Alkinyl mit 2 bis 6 Kohlenstoffatomen, Alkoxy mit 1 bis 4 Kohlenstoffatomen, Alkylthio mit 1 bis 4 Kohlenstoffatomen, Cycloalkyl mit 3 bis 6 Kohlenstoffatomen oder Halogenmethoxy stehen.

4. Verfahren gemäß Anspruch 1, **dadurch gekennzeichnet, daß** man bei der Durchführung der ersten Stufe bei Temperaturen zwischen 100°C und 200°C und bei der Durchführung der zweiten Stufe bei Temperaturen zwischen 0°C und 120°C arbeitet.

5. 1,3-Dimethyl-5-fluor-pyrazol-4-carbonsäure-fluorid der Formel

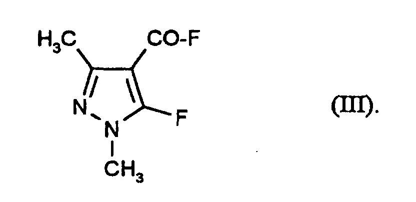

## Claims

1. Process for the preparation of 1,3-dimethyl-5-fluoro-pyrazole-4-carboxanilides of the formula 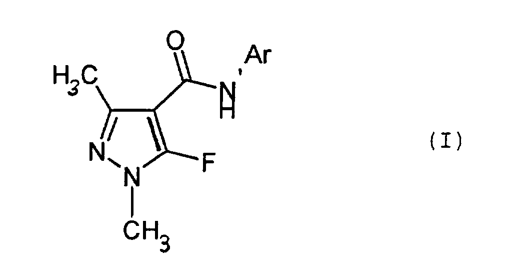 in which
Ar represents optionally substituted phenyl,
**characterized in that**
a) in a first stage, 1,3-dimethyl-5-chloro-pyrazole-4-carbonyl chloride of the formula 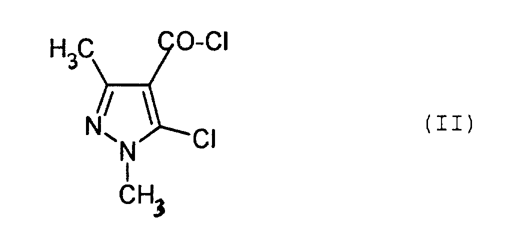 is reacted with fluorides, if appropriate in the presence of a diluent, and
b) in a second stage, the 1,3-dimethyl-5-fluoropyrazole-4-carbonyl fluoride formed in this reaction, of the formula 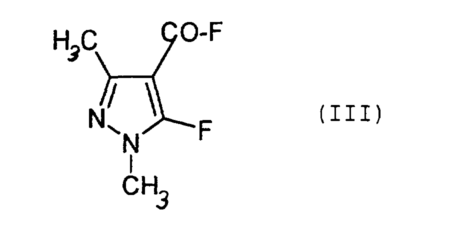 is reacted with anilines of the formula
H₂N-Ar (IV)
in which
Ar has the abovementioned meaning,
if appropriate in the presence of an acid acceptor and if appropriate in the presence of a diluent.

2. Process according to Claim 1, **characterized in that** alkali metal fluorides, ammonium fluoride or phosphonium fluorides are employed in carrying out the first stage.

3. Process according to Claim 1, **characterized in that** anilines of the formula (IV) in which
Ar represents a grouping of the formula 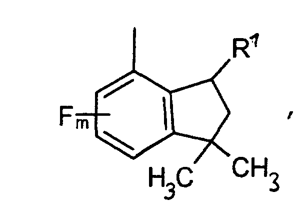 in which
m represents 0 or 1 and
R¹ represents hydrogen or methyl,
or
Ar represents a grouping of the formula 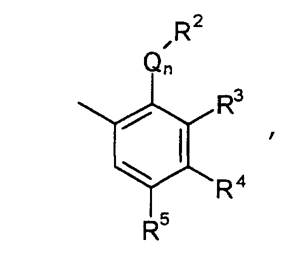 in which
n represents 0 or 1,
Q represents alkylene having 1 to 3 carbon atoms, alkenylene having 2 or 3 carbon atoms or alkinylene having 2 or 3 carbon atoms, or represents a grouping of the formula -(CH₂)ₜ-CH=, in which
t represents the numbers 0, 1, 2 or 3 and the -(CH₂)ₜ-unit is bonded to the phenyl ring,
or represents a grouping of the formula - (CH₂)ₚ-Y- (CH₂)ᵣ-, in which
p and r independently of one another represent the numbers 0, 1 or 2 and
Y represents oxygen or sulphur, the -(CH₂)ₚ-unit in each case being bonded to the phenyl ring,
R² represents cycloalkyl having 3 to 12 carbon atoms which is optionally monosubstituted or polysubstituted in an identical or different manner by alkyl having 1 to 8 carbon atoms, alkoxy having 1 to 8 carbon atoms, halogen and/or cyano, or represents cycloalkenyl having 3 to 12 carbon atoms which is optionally monosubstituted or polysubstituted in an identical or different manner by alkyl having 1 to 8 carbon atoms, alkoxy having 1 to 8 carbon atoms, halogen and/or cyano, or
represents bicycloalkyl having 6 to 12 carbon atoms which is optionally monosubstituted or polysubstituted in an identical or different manner by alkyl having 1 to 8 carbon atoms, alkoxy having 1 to 8 carbon atoms, halogen and/or cyano, or
represents oxacycloalkyl having 2 to 12 carbon atoms which is optionally monosubstituted or polysubstituted in an identical or different manner by alkyl having 1 to 8 carbon atoms, alkoxy having 1 to 8 carbon atoms, halogen and/or cyano, or
represents oxacycloalkenyl having 4 to 12 carbon atoms which is optionally monosubstituted or polysubstituted in an identical or different manner by alkyl having 1 to 8 carbon atoms, alkoxy having 1 to 8 carbon atoms, halogen and/or cyano, or
represents thiacycloalkyl having 3 to 12 carbon atoms, which is optionally monosubstituted or polysubstituted in an identical or different manner by alkyl having 1 to 8 carbon atoms, alkoxy having 1 to 8 carbon atoms, halogen and/or cyano, or
represents thiacycloalkenyl having 4 to 12 carbon atoms which is optionally monosubstituted or polysubstituted in an identical or different manner by alkyl having 1 to 8 carbon atoms, alkoxy having 1 to 8 carbon atoms, halogen and/or cyano, or
represents azacycloalkyl having 2 to 12 carbon atoms which is optionally monosubstituted or polysubstituted in an identical or different manner by alkyl having 1 to 8 carbon atoms, alkoxy having 1 to 8 carbon atoms, halogen and/or cyano,
the saturated or unsaturated ring in each case being divalent if -Q-R² represents -(CH₂)ₜ-CH=,
and
R³, R⁴ and R⁵ independently of one another represent hydrogen, halogen, cyano, alkyl having 1 to 6 carbon atoms, alkenyl having 2 to 6 carbon atoms, alkinyl having 2 to 6 carbon atoms, alkoxy having 1 to 4 carbon atoms, alkylthio having 1 to 4 carbon atoms, cycloalkyl having 3 to 6 carbon atoms or halogenomethoxy,
are employed in carrying out the second stage.

4. Process according to Claim 1, **characterized in that** the first stage is carried out at temperatures between 100°C and 200°C and the second stage is carried out at temperatures between 0°C and 120°C.

5. 1,3-Dimethyl-5-fluoro-pyrazole-4-carbonyl fluoride of the formula

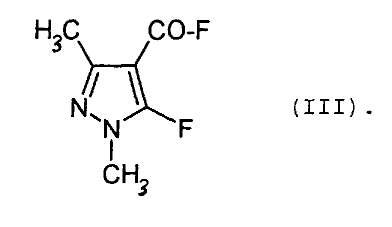

## Revendications

1. Procédé de préparation de 1,3-diméthyl-5-fluoropyrazol-4-carboxanilides de formule : 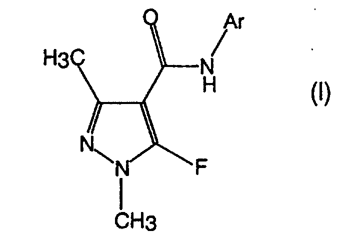 dans laquelle :
Ar représente un radical phényle éventuellement substitué,
**caractérisé en ce que** :
a) dans une première étape, on fait réagir le chlorure de l'acide 1,3-diméthyl-5-chloropyrazol-4-carboxylique de formule : 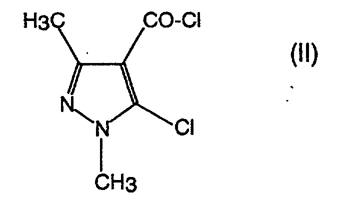 avec un fluorure, le cas échéant, en présence d'un agent de dilution, et
b) dans une deuxième étape, on fait réagir le fluorure de l'acide 1,3-diméthyl-5-fluoropyrazol-4-carboxylique ainsi obtenu de formule : 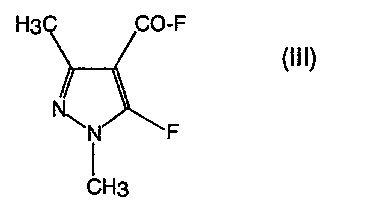 avec une aniline de formule :
H₂N - Ar (IV)
dans laquelle :
Ar a la signification indiquée ci-dessus,
le cas échéant en présence d'un accepteur d'acide et le cas échéant, en présence d'un agent de dilution.

2. Procédé suivant la revendication 1, **caractérisé en ce que** l'on met en oeuvre pour effectuer la première étape, un fluorure de métal alcalin, un fluorure d'ammonium ou un fluorure de phosphonium.

3. Procédé suivant la revendication 1, **caractérisé en ce que** l'on met en oeuvre pour effectuer la deuxième étape, une aniline de formule (IV), dans laquelle :
Ar représente un groupement de formule : 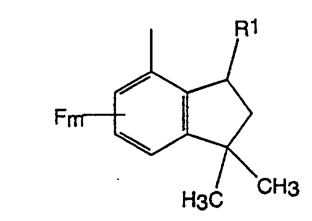 dans laquelle :
m représente 0 ou 1, et
R¹ représente l'atome d'hydrogène ou le radical méthyle, ou
Ar représente un groupement de formule : 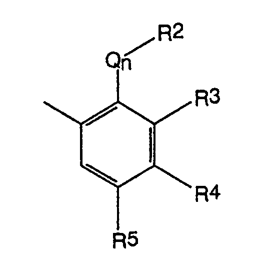
dans laquelle :
n représente 0 ou 1 ;
Q représente un radical alcoylène ayant 1 à 3 atomes de carbone, alcénylène ayant 2 ou 3 atomes de carbone ou alcynylène ayant 2 ou 3 atomes de carbone ou un groupement de formule -(CH₂)ₜ-CH=, dans laquelle :
t représente le nombre 0, 1, 2 ou 3 et l'unité -(CH₂)ₜ- est liée au cycle phényle,
ou un groupement de formule -(CH₂)ₚ-Y-(CH₂)ᵣ-, dans laquelle :
p et r représentent indépendamment l'un de l'autre, le nombre 0, 1 ou 2, et
Y représente l'atome d'oxygène ou de soufre, où l'unité -(CH₂)ₚ- est liée au cycle phényle ;
R² représente un radical cycloalcoyle ayant 3 à 12 atomes de carbone, éventuellement substitué une ou plusieurs fois, de manière identique ou différente, par un radical alcoyle ayant 1 à 8 atomes de carbone, alcoxy ayant 1 à 8 atomes de carbone, un atome d'halogène et/ou le radical cyano, ou
un radical cycloalcényle ayant 3 à 12 atomes de carbone, éventuellement substitué une ou plusieurs fois, de manière identique ou différente, par un radical alcoyle ayant 1 à 8 atomes de carbone, alcoxy ayant 1 à 8 atomes de carbone, un atome d'halogène et/ou le radical cyano, ou
un radical bicycloalcoyle ayant 6 à 12 atomes de carbone, éventuellement substitué une ou plusieurs fois, de manière identique ou différente, par un radical alcoyle ayant 1 à 8 atomes de carbone, alcoxy ayant 1 à 8 atomes de carbone, un atome d'halogène et/ou le radical cyano, ou
un radical oxacycloalcoyle ayant 2 à 12 atomes de carbone, éventuellement substitué une ou plusieurs fois, de manière identique ou différente, par un radical alcoyle ayant 1 à 8 atomes de carbone, alcoxy ayant 1 à 8 atomes de carbone, un atome d'halogène et/ou le radical cyano, ou
un radical oxacycloalcényle ayant 4 à 12 atomes de carbone, éventuellement substitué une ou plusieurs fois, de manière identique ou différente, par un radical alcoyle ayant 1 à 8 atomes de carbone, alcoxy ayant 1 à 8 atomes de carbone, un atome d'halogène et/ou le radical cyano, ou
un radical thiacycloalcoyle ayant 3 à 12 atomes de carbone, éventuellement substitué une ou plusieurs fois, de manière identique ou différente, par un radical alcoyle ayant 1 à 8 atomes de carbone, alcoxy ayant 1 à 8 atomes de carbone, un atome d'halogène et/ou le radical cyano, ou
un radical thiacycloalcényle ayant 4 à 12 atomes de carbone, éventuellement substitué une ou plusieurs fois, de manière identique ou différente, par un radical alcoyle ayant 1 à 8 atomes de carbone, alcoxy ayant 1 à 8 atomes de carbone, un atome d'halogène et/ou le radical cyano, ou
un radical azacycloalcoyle ayant 2 à 12 atomes de carbone, éventuellement substitué une ou plusieurs fois, de manière identique ou différente, par un radical alcoyle ayant 1 à 8 atomes de carbone, alcoxy ayant 1 à 8 atomes de carbone, un atome d'halogène et/ou le radical cyano,
où le cycle saturé ou insaturé est chaque fois lié deux fois lorsque -Q-R² représente -(CH₂)ₜ-CH= ;
R³, R⁴ et R⁵ représentent indépendamment l'un de l'autre, les atomes d'hydrogène, d'halogène, les radicaux cyano, alcoyle ayant 1 à 6 atomes de carbone, alcényle ayant 2 à 6 atomes de carbone, alcynyle ayant 2 à 6 atomes de carbone, alcoxy ayant 1 à 4 atomes de carbone, alcoylthio ayant 1 à 4 atomes de carbone, cycloalcoyle ayant 3 à 6 atomes de carbone ou halogénométhoxy.

4. Procédé suivant la revendication 1, **caractérisé en ce que** l'on travaille pour effectuer la première étape, à des températures comprises dans l'intervalle allant de 100°C à 200°C et pour effectuer la deuxième étape, à des températures comprises dans l'intervalle allant de 0°C à 120°C

5. Fluorure de l'acide 1,3-diméthyl-5-fluoropyrazol-4-carboxylique de formule :
